# EUROPEAN PATENT APPLICATION

(11) **EP 4 095 226 A1**
(43) Date of publication of application: **30.11.2022**
(21) Application number: 21757148.8
(22) Date of filing: 26.01.2021
(51) Int. Cl.: C12M 1/00, C12M 1/34, C12M 1/26, C12M 1/12, C12M 1/02

(54) **CELL SEPARATION APPARATUS FOR BIOREACTOR**

(30) Priority: 19.02.2020 CN 202010101731
(71) Applicant: Alit Biotech (Shanghai) Co., Ltd., Shanghai 201615 (CN)
(72) Inventor: LIU, Yu, Shanghai 201613 (CN); CHEN, Rui, Shanghai 201613 (CN)
(74) Representative: Wang, Bo
(86) International application number: PCT/CN2021/073791
(87) International publication number: WO 2021/164509

(57) **Abstract**

Disclosed in the present application is a cell separation apparatus for a bioreactor, comprising: a cell separation assembly, which is arranged within a tank body and submerged below the fluid surface of a mixture, said assembly comprising a filter membrane and a fluid guide canal, the filter membrane is constructed to filter the mixture and guide separated fluid into the fluid guide canal; the fluid guide canal comprises a radial outer side wall and a radial inner side wall, an inner empty cavity of the fluid guide canal is in communication with fluid in the tank body only by means of one or many openings on the radial inner side wall or the radial outer side wall; and a tangential flow drive assembly, which comprises one or many paddles, the paddle is located at a radial inner part of the cell separation assembly and can turn around a turning shaft, so as to drive the mixture to perform tangential circulatory flow movement along an inner side surface and an outer side surface of the cell separation assembly. The cell separation apparatus achieves low shear force and the filter membrane is not easily clogged.

## Description

### TECHNICAL FIELD

The present disclosure involves the field of bioreactors. More specifically, the present disclosure relates to a cell separation apparatus for a bioreactor.

### BACKGROUND

In animal cell expansion, more and more processes involve the separation of cells from culture medium or other liquids, comprising perfusion culture, fluid exchange, or cell cleaning. Traditional separation devices include ATF systems and TFF systems. These systems use a diaphragm pump or centrifugal pump as the power to pump the mixture of cells and culture medium into the hollow fiber column, and use the fiber pores of the hollow fiber column to separate cells and culture medium. These systems using the diaphragm pump or centrifugal pump as the power to pump the mixture of cell culture medium have the disadvantage of providing high shear force. Animal cells (especially human T cells or stem cells) are very sensitive to shear force. Excessive shear force may cause tumorigenesis of stem cells or loss of cell logo, which seriously affects the safety of cell therapy products.

Another separation device uses a filtration system floating above the liquid level of the mixture to separate cells from the culture medium. The flow direction of the mixture of the separation device is consistent with the filtration direction of the filter membrane, which is easy to block the filter membrane in the process of use. Filter membrane obstruction often means the failure of the entire process, which not only brings huge economic losses to the enterprise but also delays the precious biological samples and treatment time of patients.

### SUMMARY

One object of the present disclosure is to provide a cell separation apparatus capable of overcoming at least one defect in the prior art.

According to all aspects described below, the theme of technology is explained. For convenience, various examples of various aspects of the subject technology are described as numbered clauses (1, 2, 3, etc.). These clauses are provided as examples and do not limit the theme of technology of the present disclosure
1. A cell separation apparatus for a bioreactor, comprising a tank used for containing a mixture of cells and a liquid, wherein the cell separation apparatus comprises: a cell separation component and a tangential flow driving component; wherein
   the cell separation component is arranged in a tank and immersed below a liquid level of the mixture and include a filter membrane and a liquid guiding groove, wherein the filter membrane is configured to filter the mixture and guide a separated liquid into the liquid guiding groove, the liquid guiding groove comprises a radial outer wall and a radial inner wall, and an internal cavity of the liquid guiding groove is only in fluid communication with the tank through one or more openings on the radial inner wall and through the filter membrane, or only in fluid communication with the tank through one or more openings on the radial outer wall and through the filter membrane; and
   the tangential flow driving component comprises one or more blades positioned radially inside the cell separation component and capable of rotating about a rotation axis to drive the mixture to flow tangentially along the filter membrane.
2. The cell separation apparatus according to claim 1, further comprising a liquid collection component arranged outside the tank, the liquid collection component comprising a collection container in fluid communication with the liquid guiding groove.
3. The cell separation apparatus according to claim 2, wherein the liquid collection component comprises a collection tube in fluid communication with the collection container to the liquid guiding groove.
4. The cell separation apparatus according to claim 3, wherein a pump is set on the collection tube, and the separated liquid is pumped from the liquid guiding groove to the collection container.
5. The cell separation apparatus according to claim 4, wherein the pump comprises a peristaltic pump.
6. The cell separation apparatus according to the claim 3, wherein a pressure sensor is set in the collection tube to monitor a liquid pressure in the collection tube.
7. The cell separation apparatus according to any one of claims 1-6, wherein the cell separation component further comprises a support sheet positioned between the liquid guiding groove and the filter membrane, and the support sheet is configured to support the filter membrane.
8. the cell separation apparatus according to claim 7, wherein the filter membrane is fixed to the support sheet by hot melting, cohesion or laser welding.
9. The cell separation apparatus according to claim 7, wherein the support sheet is connected to the liquid derivation groove through hot melting, a concave-convex match or laser welding.
10. The cell separation apparatus according to claim 3, wherein the liquid guiding groove is provided with a guiding tube connected with the collection tube.
11. The cell separation apparatus according to claim 10, wherein the guiding tube extends vertically upward from a top wall of the liquid guiding groove.
12. The cell separation apparatus according to any one of claims 1-6, wherein the liquid guiding groove is provided with a support column configured to support the cell separation component above the bottom of the tank and separate the cell separation component from the bottom of the tank.
13. The cell separation apparatus according to claim 12, wherein the support column is configured to be connected to the radial inner wall of the liquid guiding groove through a radial radiation arm.
14. The cell separation apparatus according to claim 12, wherein the support column is configured to extend downward from a bottom wall of the liquid guiding groove.
15. The cell separation apparatus according to any one of claims 1-6, wherein the filter membrane is made of PTFE, PP, PC, nylon, PES or sintered porous material.
16. The cell separation apparatus according to any of claims 1-6, wherein the aperture diameter of the filter membrane is about 5 µm, about 10 µm, about 20 µm, about 50 µm, about 100 µm or about 200 µm.
17. The cell separation apparatus according to claim 7, wherein the support sheet is made of soft materials with holes.
18. The cell separation apparatus according to claim 17, wherein the soft materials comprise soft glue materials.
19. The cell separation apparatus according to claim 17, wherein the shape of the holes is approximately circular, approximately square or approximately hexagonal.
20. The cell separation apparatus according to claims 7, wherein the support sheet is made of fibrous porous material.
21. The cell separation apparatus according to claim 7, wherein the thickness of the support sheet is between 50 µm-300 µm.
22. The cell separation apparatus according to any one of claims 1-6, wherein the tangential flow driving component further comprises a motor for driving the rotation of the blades.
23. The cell separation apparatus according to any one of claims 1-6, wherein the diameter of the blades is set to be slightly smaller than an inner diameter of the cell separation component to provide an effect of tangential flow in a narrow space.
24. The cell separation apparatus according to any one of claims 1-6, wherein a diameter of the blades is configured as 30%-45% of an inner diameter of the tank.
25. The cell separation apparatus according to any one of claims 1-6, wherein a distance between a radial outermost point of the blades and a radial inner surface of the filter membrane is 1 mm-5 mm.
26. The cell separation apparatus according to any one of claims 1-6, wherein the blades are in the form of a full axial flow blades.
27. The cell separation apparatus according to any one of claims 1-6, wherein the blades are in the form of single-layer or multi-layer elephant ear blades, marine blades, or helical blades.

Other features and advantages of the subject technology of the present disclosure would be described in the following description, and would be apparent in part from the description, or may be learned by practicing the subject technology of the present disclosure. The advantages of the subject technology of the present disclosure would be realized and obtained through the structure specially pointed out in the written description, the claims and the drawings.

It should be understood that both the foregoing general description and the following detailed description are exemplary and illustrative, and are intended to provide a further description of the subject technology of the present disclosure.

### BRIEF DESCRIPTION OF THE DRAWINGS

After reading the specific implementation of the following drawings, it would better understand the multiple aspects of this disclosure, in the attachment:
FIG. 1 is a schematic diagram of the cell separation apparatus of the present disclosure;
FIG. 2 is a local section view of the cell separation component of the cell separation apparatus shown in FIG.1;
FIG. 3 is a section of cell separation components of the cell separation apparatus shown in FIG.1; and
FIG. 4 and FIG. 5 are schematic diagrams of support sheets of the cell separation apparatus shown in FIG. 1.

### DETAILED DESCRIPTION

The following would be referred to the picture to describe the present disclosure, and the attachment shows several embodiments of this disclosure. However, it should be understood that the present disclosure can be presented in a variety of different ways, and it is not limited to the implementation example described below; In fact, the implementation example described below aims to make the disclosure more complete, and fully explain the scope of protection of the present disclosure to the technical personnel of the art. It should also be understood that the implementation examples of this article can be combined in various ways to provide more additional embodiments.

It should be understood that in all the attachments, the same attachment indicates the same components. In the attachment, for a clear time, the size of some characteristics can be deformed.

It should be understood that the use of words in the manual is only used to describe specific embodiments, and it is not designed to limit the present disclosure. All terms (including technical terms and scientific terms) used in the manual (including technical terms and scientific terms) have the meaning of the general understanding of technicians in the art. For simplicity and/or clarity, the functions or structures of public knowledge can no longer be explained in detail.

The single-number forms of the terms "one," "what," and "the should" are used to include plural forms unless clearly indicated. The use of the terms "including," "include," "containing," and "containing" represent the characteristics of the presence, but do not reject one or more other characteristics. The use of the terms "and/or" include any or more combinations of one or more in the relevant list. The terms use "between X and Y" and "between about X and Y" should be interpreted as including X and Y. The use of the term "between approximately X and Y" means "between about X and about Y", and the use of "from X to Y" means "from about X to about Y."

In the specification, when a component is described as located "on" another component, "attached" to another component, "connected" to another component "coupled" to another component, or "contacting" another component, etc., this component can be directly located on another component, attached to another component, connected to another component, or coupled to another component or contacting another element, and intermediate components can exist. In contrast, when a component is "directly" located "on" another component, "directly attached" to another component, "directly connected" to another component, "directly coupled" to another component or "directly contacting" another part, there would be no intermediate components. In the specification, one feature arrangement is "adjacent" to another feature, which can refer to one feature that has a part that overlaps with adjacent features or is located above or below the adjacent features.

In the specification, the use of "upper," "down," "left," "right," "front," "back," "high," "low," and other terms related to spatial relations can explain the relationship of one feature and another feature in the drawings. It should be understood that in addition to the orientation of the attached picture, the spatial relationship also includes the different orientations of the device in practical uses or operations. For example, when the device is inverted in the attached figure, it was originally described as the features of other features "below", and at this time, it can be described as "above" of other features. The device can also be directed in other ways (rotating 90 degrees or in other directions), and the relative spatial relationship would be explained accordingly.

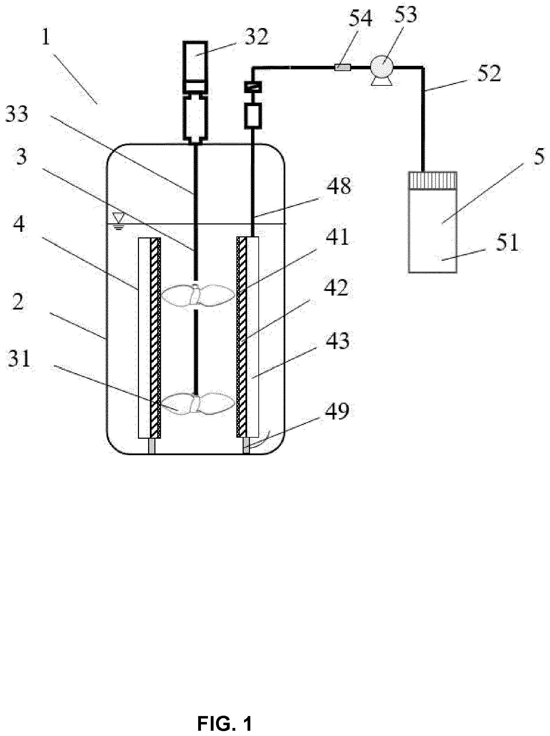

FIG.1 shows the cell separation apparatus 1 according to some embodiments of the present disclosure. The cell separation apparatus 1 is used for small disposable bioreactors. The disposable bioreactor includes a tank 2 to accommodate a mixture of cells and a medium, or a mixture of cells and other liquids. As shown in the figure, cell separation apparatus 1 includes a tangential flow driving component 3, a cell separation component 4, and a liquid collection component 5. The tangential flow driving component 3 and the cell separation component 4 can be placed in the bioreactor in the tank 2, and the liquid collection component 5 can be placed in the bioreactor and located outside the tank 2. The tangential flow driving component 3 is configured to drive the mixture of cells and the medium (or other liquids) flow circularly along the cell separation component 4 along a tangential direction; the cell separation component 4 is configured to separate the cells from the medium (or other liquids) during the cyclic flow of the mixture; and the liquid collection component 5 is used to collect the medium or other liquid separated from cells.

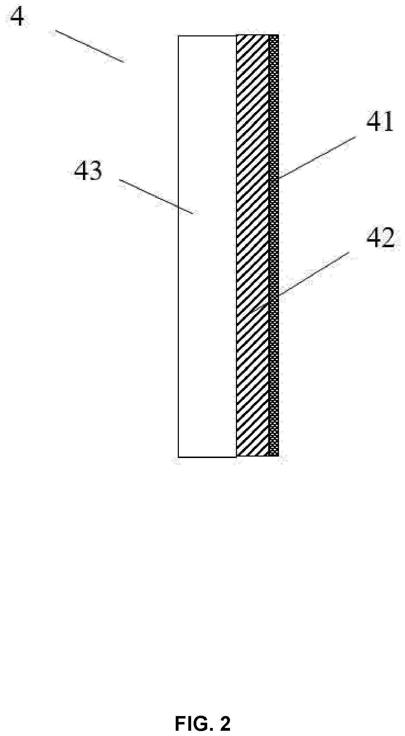

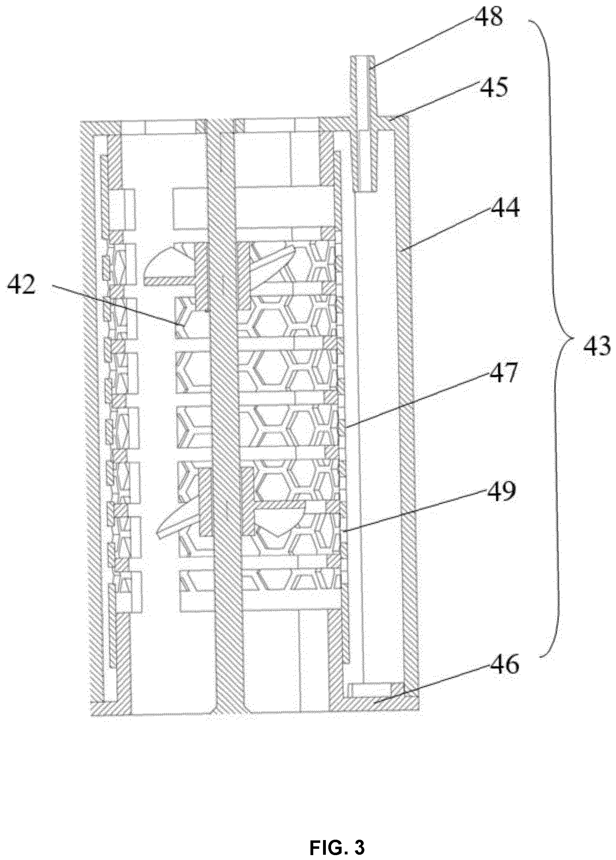

The cell separation component 4 is placed in the tank 2, and the liquid surface of the mixture of the cell and the medium (or other liquid) is immersed. The cell separation component 4 is roughly a hollow cylinder, and includes a filter membrane 41, a support sheet 42, and a liquid guiding groove 43 that are adjacent and connected to each other, as illustrated in FIGs. 2 and 3. The filter membrane 41 is used to filter cells and the medium (or other liquids) mixtures. While blocking cells, the medium (or other liquid) flows to the liquid guiding groove 43 through the filtering film (or other liquid) while using the filter membrane 41. The support sheet 42 is used to support the filter membrane 41 to keep the filter membrane 41 flat. The liquid guiding groove 43 is used to accommodate the medium (or other liquid) of the filter membrane 41 and guide the medium to flow out of the tank 2.

As shown in FIG. 3, the liquid guiding groove 43 shows the middle empty tube, and includes a radial outer wall 44 and a radial inner wall 47, and a top wall 45 and a bottom wall 46 extending between the radially outer wall 44 and the radially inner wall 47. The radial outer wall 44, the radial inner wall 47, the top wall 45 and the bottom wall 46 surround the internal cavity of the liquid guiding groove 43, and the internal cavity is only in fluid communication with the tank 2 through one or more openings 49 on the radial inner wall 47 and through the filter membrane 41.

The liquid guiding groove 43 also has a guiding tube 48 used for guiding the medium (or other liquid) contained in the liquid guiding groove 43 to flow out of the liquid guiding groove 43. The guiding tube 48 can extend vertically from the top wall 45 of the liquid guiding groove 43, such as extending to the liquid surface of the mixture of the cell and the medium (or other liquid).

The mixture of the medium (or other liquid) can circulate through the gap below the cell separation component 4. The support column 49 can extend down from the bottom wall 46 of the liquid guiding groove 43. In other embodiments, the support column 49 may be located in the hollow tube of the liquid guiding groove 43, and is connected by the radial inner wall 47 of the radial drainage slot 43 from the radial inner wall 47 from its radial radiation arm. In some embodiments, the height design of the support column 49 makes the gap between the cell separation component 4 and the tank 2 as small as possible, so as not to cause turbulence to affect cell growth.

The filter membrane 41 can be made of PCTE, PETE, PTFE, PP, PC, Nylon, PES or sintering porous materials. The filter membrane 41 is treated with hydrophilic and positive charge or negative charge, so it is not easy to be adsorbed by cells to block the filter membrane. The filter membrane 41 Followed by biotechnology (such as stem cells, tumor cells, CHO cells, or microcarriers, etc.), a variety of pores can be used (including about 5 µm, about 10 µm, about 20 µm, about 50 µm, about 100 µm, about 200 µm, etc.)

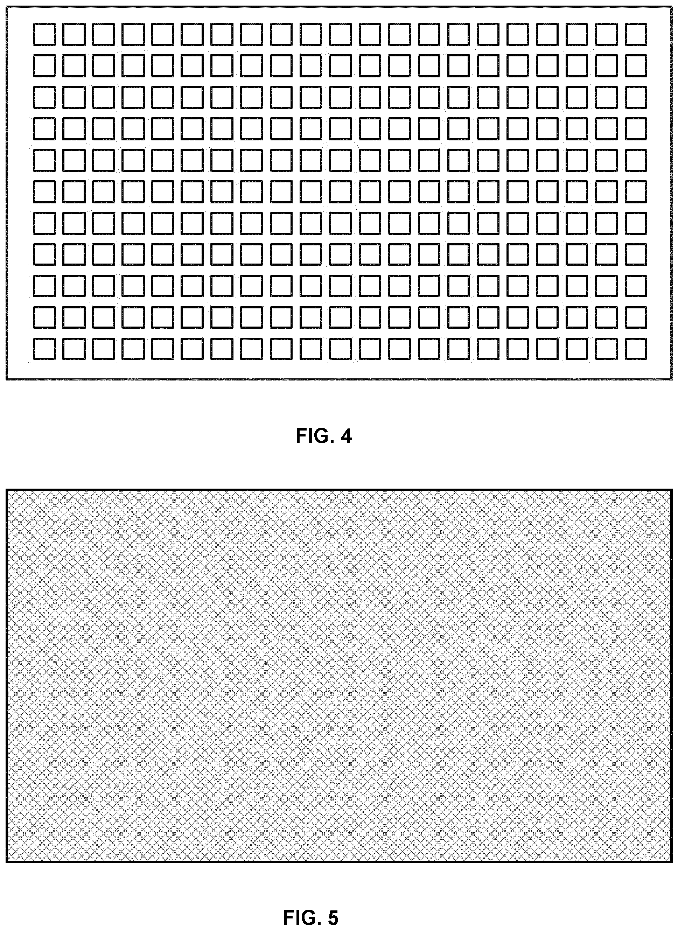

The support tablets 42 can be made of punching soft materials (eg, soft glue material), as shown in FIG. 4. The shape of the holes can be roughly circular, general square, rough hexagonal, or any other shape. In some embodiments, the support tablets 42 can also be made of fiber porous materials, as shown in FIG. 5. The thickness of the support tablets 42 can be between about 50 µm - about 300 µm.

The filter membrane 41 is fixed to the support tablets 42 by means of hot melting, cohesion, laser welding, etc., and the support tablets 42 is connected to the radial inner wall 47 of the liquid guiding groove 43 by means of hot melting, concave convex fitting, laser welding, etc.

In some embodiments, the radial outer wall 44, the radial inner wall 47, the top wall 45, and the bottom wall 46 of the liquid guiding groove 43 surround the internal cavity of the liquid guiding groove 43, and the inner cavity is only in fluid communication with the tank 2 through one or more openings on the radial outer wall 44 (rather than the radial inner wall 47) and through the filter membrane 41. The filter membrane 41 is fixed to the support tablets 42 by means of hot melting, cohesion, laser welding, etc., and the support sheet 42 is connected to the radial outer wall 44 of the liquid guiding groove 43 by means of hot melting, concave convex fitting, laser welding, etc.

The tangential flow driving component 3 includes one or more paddles 31 and a motor 32 for driving paddles 31. The paddles 31 is placed in the tank 2 and is immersed in a mixture of cells and the medium (or other liquids). The paddle 31 may be located in the radial interior of the cell separation component 4 and rotate around the rotation axis 33 to drive the mixture of cells and culture medium (or other liquid) to circulate tangentially (i.e., vertical direction) along the inner and outer surfaces (for example, including filter membrane 41, radial outer wall 44, radial inner wall 47 of liquid guiding groove 43, etc.) of the cell separation component 4. The rotation of paddle 31 would not cause too high shear force, so it would not cause damage to animal cells. The motor 32 is placed outside the tank 2, and its driving shaft is directly connected to the rotation axis 33 of the paddles 31, or the rotation axis 33 of the paddles 31 is connected through the speed adjustment mechanism such as the gearbox to drive the rotation axis 33 and the paddles 31 rotation. The motor 32 may be connected to the controller (not shown).

The output power of the motor 32 is one of the key design parameters. The larger the output power of the motor 32, the greater the flowing speed of the mixture, and the longer the service life of the filter membrane 41. Especially in the case of high-density cell culture, increasing the increase in flowing speed can reduce the blockage speed of the filter membrane 41.

The distance between the blades 31 and the filter membrane 41 is also one of the key design parameters. The closer the distance, the higher the velocity of the liquid in the mixture passing through the filter membrane 41, but it would also significantly increase the local shear force of the liquid, which would affect the physiological characteristics of stem cells. The diameter of the blades 31 is set to be less than the inner diameter of the filter membrane 41 (for example, the distance between the radial most of the radial blades 31 and the radial surface of the filter membrane 41 is about 1 mm-about 5 mm), provide the flowing effect of tangential flow in a small space. In some embodiments, the diameter of the blades 31 can be designed into about 30%-45% of the inner diameter of the tank. The blades 31 can be used in the form of a complete axis, such as single-layer or multi-layer elephant ear blades, marine blades and helical blades.

The liquid collection component 5 is placed outside the tank 2. The liquid collection component 5 includes collection container 51 and collection tube 52. The collection tube 52 is connected to the collection container 51, and the other end is connected to the guiding tube 48 to the liquid derivatives 43 to guide the medium (or other liquid) of the liquid derivative groove 43 to the collection container 51. In some embodiments, the collection tube 52 can be flexible, so that the collection container 51 can be placed in any appropriate location outside the tank 2. A pump 53 (such as peristaltic pump, etc.) may be set on the collection pipe 52 and connected to the controller to help the medium (or other liquid) pump and pump to the collection container 51. The controller can change the pump speed change of the pump 53 of the signal control pump 53 according to the process requirements, the pump speed of the other replenishment pump, the weight of the pump, or some sensors (such as the living cell sensor) to adjust the drainage rate.

In some embodiments, the pressure sensor 54 connected to the controller can be set on the collection tube 52 to monitor the liquid pressure in the collection tube 52. When the pressure sensor 54 detects the cell blocking the filter membrane 41 and the pressure in the collection tube 52 rises, the controller stops the work of the pump 53 and sends the alarm signal to the operator.

The operation method of the cell separation apparatus 1 is introduced below. First, put the bioreactor into the ultra-net platform for adding the medium and for inoculation, and then place the bioreactor to the console, and connect the corresponding sensor. The controller sends a start signal to the motor 32, and the motor 32 drives the blades 31 to rotate around the rotation shaft 33, thereby driving the mixture of cells and culture medium (or other liquids) to circulate in a tangential (i.e., vertical) direction along the inner and outer surfaces (including, for example, the filter membrane 41, the radial outer wall 44 of the liquid guiding groove 43, the radial inner wall 47, etc.) of the cell separation component 4. When you need to change the liquid, clean these components, or separate cells or microcarriers, the controller starts the pump 53. The pumping effect generated by the pump 53 enables the medium (or other liquid) to separate the cells from the liquid in filter membrane 41 and enter the liquid guiding groove 43, and then enter the collection container 51 by collection tube 52.

After discharging a certain volume of abandoned medium from the tank 2, the pump 53 can reverse the operation, so that the liquid in the collection tube 52 can flow reversely and rush to the filter membrane sheet 41 to prevent the cells from blocking the filter membrane 41.

The cell separation apparatus 1 according to the embodiments of the present disclosure uses the blades 31 to drive the mixture, so that a low shear force can be achieved and the cells are not affected.

In addition, the cell separation apparatus 1 according to the embodiments of the present disclosure filter the mixture through the tangential flow of the mixture along the filter membrane, so that the filter membrane is not easily blocked and can be used for a long time.

Although the present disclosure embodiments have been described, the technical personnel of the art should understand that in the case of essentially not separated from the spirit and scope of the present disclosure, multiple changes can be made on the present disclosure demonstration embodiments. Therefore, all changes and changes are included within the scope of the present disclosure limited by claims. The present disclosure is limited by additional claims, and equivalent of these claims is also included.

## Claims

1. A cell separation apparatus for a bioreactor comprising a tank used for containing a mixture of cells and a liquid, wherein the cell separation apparatus comprises: a cell separation component and a tangential flow driving component, wherein
the cell separation component is arranged in a tank and immersed below a liquid level of the mixture and include a filter membrane and a liquid guiding groove, wherein
the filter membrane is configured to filter the mixture and guide a separated liquid into the liquid guiding groove,
the liquid guiding groove comprises a radial outer wall and a radial inner wall, and an internal cavity of the liquid guiding groove is only in fluid communication with the tank through one or more openings on the radial inner wall and through the filter membrane, or only in fluid communication with the tank through one or more openings on the radial outer wall and through the filter membrane; and
the tangential flow driving component comprises one or more blades positioned radially inside the cell separation component and capable of rotating about a rotation axis to drive the mixture to flow tangentially along the filter membrane.

2. The cell separation apparatus according to claim 1, further comprising a liquid collection component arranged outside the tank, the liquid collection component comprising a collection container in fluid communication with the liquid guiding groove.

3. The cell separation apparatus according to claim 2, wherein the liquid collection component comprises a collection tube in fluid communication with the collection container to the liquid guiding groove.

4. The cell separation apparatus according to claim 3, wherein a pump is disposed on the collection tube, and the separated liquid is pumped from the liquid guiding groove to the collection container.

5. The cell separation apparatus according to claim 4, wherein the pump comprises a peristaltic pump.

6. The cell separation apparatus according to claim 3, wherein a pressure sensor is disposed in the collection tube to monitor a liquid pressure in the collection tube.

7. The cell separation apparatus according to any one of claims 1-6, wherein the cell separation component further comprises a support sheet positioned between the liquid guiding groove and the filter membrane, and the support sheet is configured to support the filter membrane.

8. the cell separation apparatus according to claim 7, wherein the filter membrane is fixed to the support sheet by hot melting, cohesion or laser welding.

9. The cell separation apparatus according to claim 7, wherein the support sheet is connected to the liquid derivation groove through hot melting, a concave-convex match or laser welding.

10. The cell separation apparatus according to claim 3, wherein the liquid guiding groove is provided with a guiding tube connected with the collection tube.

11. The cell separation apparatus according to claim 10, wherein the guiding tube extends vertically upward from a top wall of the liquid guiding groove.

12. The cell separation apparatus according to any one of claims 1-6, wherein the liquid guiding groove is provided with a support column configured to support the cell separation component above the bottom of the tank and separate the cell separation component from the bottom of the tank.

13. The cell separation apparatus according to claim 12, wherein the support column is configured to be connected to the radial inner wall of the liquid guiding groove through a radial radiation arm.

14. The cell separation apparatus according to claim 12, wherein the support column is configured to extend downward from a bottom wall of the liquid guiding groove.

15. The cell separation apparatus according to any one of claims 1-6, wherein the filter membrane is made of PTFE, PP, PC, nylon, PES or sintered porous material.

16. The cell separation apparatus according to any of claims 1-6, wherein an aperture diameter of the filter membrane is about 5 µm, about 10 µm, about 20 µm, about 50 µm, about 100 µm or about 200 µm.

17. The cell separation apparatus according to claim 7, wherein the support sheet is made of soft materials with holes.

18. The cell separation apparatus according to claim 17, wherein the soft materials comprise soft glue materials.

19. The cell separation apparatus according to claim 17, wherein the shape of the holes is approximately circular, approximately square or approximately hexagonal.

20. The cell separation apparatus according to claims 7, wherein the support sheet is made of fibrous porous material.

21. The cell separation apparatus according to claim 7, wherein the thickness of the support sheet is between 50 µm-300 µm.

22. The cell separation apparatus according to any one of claims 1-6, wherein the tangential flow driving component further comprises a motor for driving the rotation of blades.

23. The cell separation apparatus according to any one of claims 1-6, wherein the diameter of the blades is set to be slightly smaller than an inner diameter of the cell separation component to provide an effect of tangential flow in a narrow space.

24. The cell separation apparatus according to any one of claims 1-6, wherein a diameter of the blades is configured as 30%-45% of an inner diameter of the tank.

25. The cell separation apparatus according to any one of claims 1-6, wherein a distance between a radial outermost point of the blades and a radial inner surface of the filter membrane is 1 mm-5 mm.

26. The cell separation apparatus according to any one of claims 1-6, wherein the blades are in the form of a full axial flow blades.

27. The cell separation apparatus according to any one of claims 1-6, wherein the blades are in the form of single-layer or multi-layer elephant ear blades, marine blades, or helical blades.
